# EUROPEAN PATENT APPLICATION

(11) **EP 3 320 833 A1**
(43) Date of publication of application: **16.05.2018**
(21) Application number: 17425116.5
(22) Date of filing: 08.11.2017
(51) Int. Cl.: A61B 5/00, G01N 33/68

(54) **METHODS FOR THE QUANTITATIVE ANALYSIS OF HUMAN BREAST MILK**

(30) Priority: 15.11.2016 IT 201600115242
(71) Applicant: Omini, Gabriele, 20064 Gorgonzola (MI) (IT)
(72) Inventor: Perri, Mariarita, 87027 Paola (CS) (IT); Cannataro, Roberto, 87040 Luzzi (CS) (IT); Omini, Gabriele, 20064 Gorgonzola (MI) (IT)

(57) **Abstract**

The object of the present invention are mehods suitable for the quantitative determination of breast milk. Our studies in human breast milk showed a close correlation between a specific biomarker and quantitative composition of breast milk. In particular, the amount of PhosphoPerilipin (P-LAP) is inversely proportional to the quantity of breast milk produced during the circadian rhythm. Therefore, it is claimed a method involving the dosage of p-LAP, also in combination with other biomarkers, suitable for the daily determination of the composition of breast milk.

## Description

### Application field:

According to the World Health Organization (www.who.int), breastfeeding is the most natural and healthy way to provide infants nutrition, all the nutrients they need to grow and to ensure their proper physiological development. Generally, all mothers are able to breastfeed but they must receive accurate informations, family support, and an adequate healthcare system in a delicate moment of their life. These themes are highlighted by WHO that point out the importance of proper information on nursing infants. It is quite natural for mothers to be anxious about their child's growth, so they need to be comforted and prepared to face up the sensitive period of breastfeeding. The fundamental point for the new life of a women is the possibility to produce unbalanced milk quantities. Therefore, a mother wonder: Is it my milk secreted in sufficient quantity and adequate for the nutritional needs necessary for my baby's growth? In this context, it is necessary to provide for every mother a simple and valid method to check the quality and quantity of daily milk production. Therefore, we have thought to develop a medical device capable of monitoring some of the biological parameters present in breast milk to improve the integration of missing milk and the psychophysical well-being of the mothers.

### SUMMARY OF THE INVENTION:

The object of the present invention is to provide a simple and convenient medical device suitable for daily quantitative determination of maternal milk.

### State of the art:

Today, less than 40% of infants are suckled with breast milk. The aim of WHO is to reach at least 50% of breastfeeding infants by 2025 declaring what following: *"If all new-borns were breast-fed by the first hour of life and such breastfeeding continued for up to 2 years, about 800,000 new-born babies would be saved each year."* It is now acclaimed that breastfeeding contributes to better mouth shape, protecting the unborn against respiratory tract disease and asthma, otitis, diarrhea, and decreasing the risk of developing in adulthood disease such as diabetes and metabolic syndrome. Although all these benefits, mom often decides not to breastfeed their babies for inadequacy feeling that their milk is not enough and /or do not meet the new-born's nutritional needs. Currently, there are not quick and simple methods daily used to evaluate qualitative and monitoring quantitative breast-milk. The empirical method widely used is to weight baby's body (after each suckling) and weight gain after a couple of days and/o weeks. Such methods are not able to ensure if baby is properly fed to mothers fostering the worries by mothers that under-nutrition can cause nutritional deficiencies resulting in permanent neuro-physical damage. Alternatively, mothers can express their breastmilk by both, manually or with electrical pumps. In this way, they know exactly the volume, which is administrated with a bottle to the baby but, although this procedure can be feasible, is very uncomfortable and normally not preferred by mothers. In fact, expressing breastmilk is must do by mothers in exceptional situations (i.e infant hospitalization). Besides that, the practice of expressing breastmilk, bypass the suckling of the infant leading to a physiological decrease in milk production. The purpose of the present invention is to provide a simple and immediate device that allows the evaluation of the quantitative/qualitative production of breastmilk by minimizing mothers' anxiety and worries for new-borns proper feeding and favouring the practice of natural nursing.

Definitions: below are the definitions of the main terms used in the present invention:
1) Phospho-Perilipine, PhosphoPerilipin (p-LAP), belongs to a class of proteins identified in eukaryotic species of vertebrate, infectious and amoebae. Composition and structure are phylogenetically highly conserved and have a high distribution for tissues containing neutral lipids (adipocytes) (AR Kimmel et al J Lipid Res. 2010 Mar; 51 (3): 468-71), including human milk. In the general term Perilipine, Perilipine in the present invention identifies all the components of the family and in particular: perilipine 1 identified by National Center for Biotechnology Information (NCBI) with Enterez GeneID: 5346; perilipine 2 GeneID: 123; perilipine 3 Enterene GeneID: 10226; perilipine 4 GeneID: 729359; Perilipine 5 Enterene GeneID: 440503. Phospho-Perilipine, PhosphoPerilipin (p-LAP) refers to all of the above-described components subjected to phosphorylation, i.e. addition of phosphate ions (PO43-) to the chain protein.
2) Sandwich Immunoassay: it is a biochemical technique, in which the protein (biomarker) is in solution (urine and blood sample), in our case breast milk, and it can be detected by the use of a specific antibody (or aptamer) attached to a matrix. Visualization is due to the gold particle shift for capillarity.
3) Western Blot: biochemical technique in which a selected protein (biomarker) is fixed to a matrix. The protein is detected with a specific antibody when this is quantitatively too low to be visualized with other techniques.The result is impressed on a photographic film.
4) Densitometric analysis: it is an analysis derived from the western blot and involves the use of dedicated software to acquire the number of pixels of the band.
5) microRNAs: are specific biomolecules which the expression (profile) describes a physiological condition (as it is in our case) or a pathological condition. To this class belong: miR21 also known as hsa-mir-21 or miRNA21, miR146a (MIR146), miR155 (MIR155) and miR16 also known as hsa-miR-16-5p.
6) Aptamer: it is a stable synthetic molecule of RNA or DNA that have specificity and selectivity for the biomarker to be identified.

### Description:

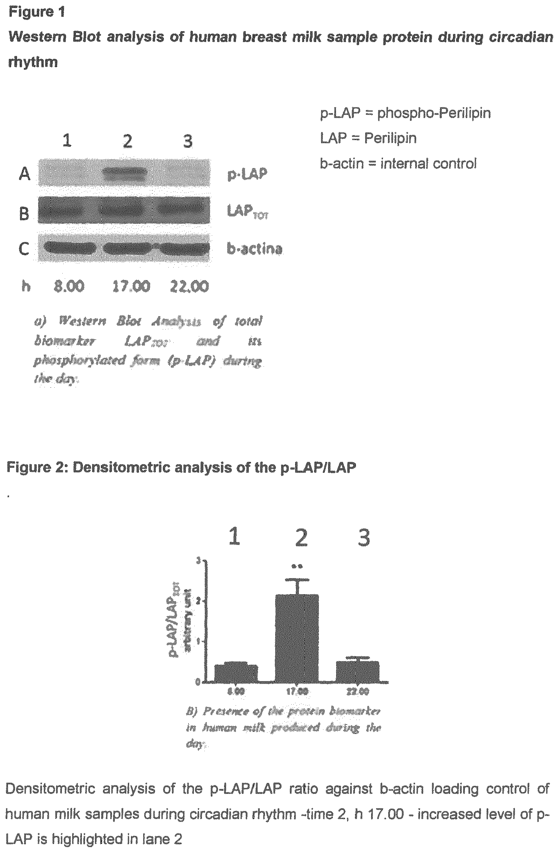

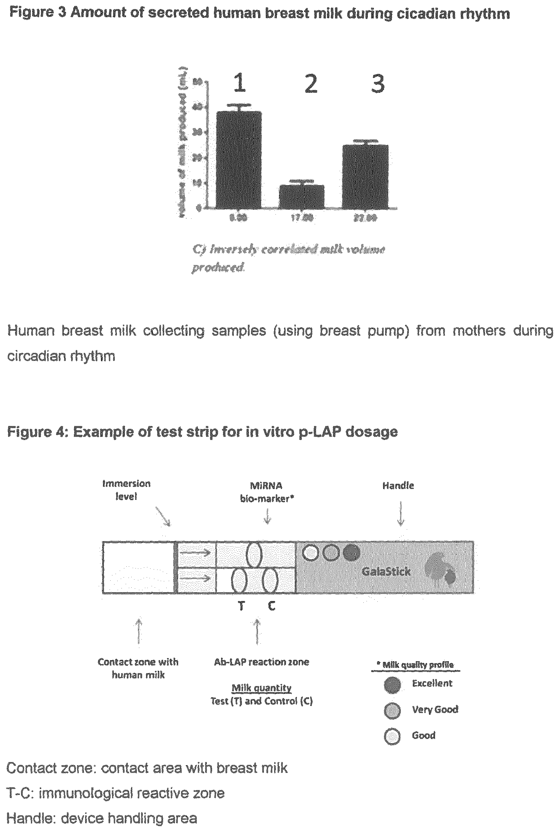

Our studies on breast milk demonstrated a close correlation between a specific biomarker and the quantitative/qualitative composition of breast milk. In particular, the presence of p-LAP is inversely proportional to the milk amount produced during the circadian rhythm. The p-LAP protein is strongly associated with cytoplasmic sites of lipid deposits in adipocytes. Under basal conditions, p-LAP is able to inhibit lipolysis. The phosphorylation of LAP mediated by c-AMP results in lipases inhibition and release of fatty acids (PM McDonough et al. PLoS One, 2013; 8 (2): e55511). As shown in Example 1, our studies have shown a close correlation among the quantity of p-LAP determined in breast milk and the amount of this protein produced during the circadian rhythm. As shown in Figure 1, western blot analysis of milk proteins, taken at different daily times, indicates the presence of p-LAP in time 2 (17.00) whereas in other selected time points, 1 and 3 (8.00 and 22.00 respectively), p-LAP has not been highlighted. Similarly, densitometric analysis of p-LAP confirmed the significantly higher expression in time 2 compared to times 1 and 3 (Figure 2). The high presence of p-LAP (time 2) corresponds to a significantly lower maternal milk level than time 1 and 3 (Figure 3). These results indicate that the p-LAP dosage in breast milk is a unique and reliable biomarker to predict the amount of breast milk. Quantification of the p-LAP biomarker can be performed using different methods. One of the most advantageous methods is the use of immunoassay, commonly known by the scientific community as a sandwich immunoassay, of serine 497 p-LAP. Example 2 briefly explain the method described above. The methodology described in Example 2 can easily be transferred to a device that can allow its industrialization. Example 2 discloses the main features of a device suitable for the measurement of p-LAP and/or its metabolites or derivatives suitable for the purpose of the present invention. In particular, the use of nitrocellulose or polyvinylidene fluoride membranes (PVDF), such as a supportive material for the immunological assay, is particularly preferred in the present invention. This material has absorption and washing characteristics that achieve a perfect compromise between the specific technical requirements and the cost and availability on the market. Methods for quantification of p-LAP and/or its isomers or derivatives chemically and/or biologically usable and claimed by the present invention are also: serine 522 p-LAP and aptamers. Similarly, devices of any size and configuration suitable for daily use in a domestic and/or professional environment such as medical, hospitals and nursing homes, outpatient clinics, research centers, and so on, may be suitably made and fall within the claims of the present invention. In Example 3, a p-LAP dosage assay can be schematically used in vitro. In Example 3 an in vitro medical device, that could be used for p-LAP dosing, was schematically reported. If desired, it is also claimed in the present invention the use in combination with other methods suitable for the immediate determination of breast milk components such as microRNAs, casein and whey proteins to determine, respectively, the modulating immune capacity and the nutritional value of the analysed human milk samples.

### EXAMPLE 1-

The p-LAP biomarker was identified in breast milk obtained by 35 women breastfeeding. As shown in Figure 2, the Western blot analysis was performed using the methodology described below (briefly: it is a method that allows the transfer of proteins from a polyacrylamide gel to a nitrocellulose membrane in order to carry out a detailed analysis of a single protein. The technique involves the use of primary antibody directed against the protein of interest and secondary antibodies capable of recognizing the constant portion of the IgG molecule used as the primary antibody. The secondary antibody is labeled with an enzyme that is recognized once linked to the primary antibody). Western blot analysis showed the presence of significant amounts of p-LAP in breast milk in exact correspondence to the minimum quantities of breast milk as shown in Figure 4. Likewise, the use of the densitometric method to highlight the biomarker proteins shows the same negative correlation already found with western blot analysis between p-LAP and the amount of breast milk. The use of microRNAs such as miR21, miR146a, miR155, miR16, which have immune modulating properties, is also claimed and allow the qualitative evaluation of the immunological profile of breast milk. These microRNAs will be visualized with phosphorescent synthetic oligonucleotides.

### EXAMPLE 2-

The device may consist of a support strip for immunological assay; this support strip is usually covered with appropriate antibodies through a chemical-physical absorption system obtained, for example, by ionic bonds, van der Waals forces, electrostatic bonds, and any other chemical-physical means suitable for the purpose. Colloidal gold particles are coated with specific antibodies. The immobilized gold-antibody complexes are able to react with the biomarker, such as p-LAP, and through a series of steps in the different zones, usually 3, of the support strip cause a color variation. Particularly when a small dose of breast milk is added to the support strip, a dissolution of gold particles occurs. If milk sample contains sufficient amounts of p-LAP, this protein is recognized by its specific antibody, carried by capillary, releasing in an equally proportional manner equivalent amounts of gold particles that in turn produce color-coded variations easily observable and therefore in ability to determine the effectiveness of the test. The amount of colloidal gold not bound to the specific antibodies is used as a positive test control.

### EXAMPLE 3:

Figure 4 shows an example of suitable device for the quantitative/qualitative measurement of breast milk. The device may consist of a simple stick composed of a solid microcellulose adsorbent support anti-p-LAP antibody with a molecular weight of about 55 kDa and in any case between 35 kDa and 75 kDa. The microcellulose or other suitable agent may have a porosity of between 0.25 and 0.95 nm, in particular 0.45 nm. Gold colloidal particles having dimensions between 0.3 and 0.9 nm, particularly 0.60 nm, are added to the stick. The T and C stick zones, as shown in Figure 4, represent the reactive zone between p-LAP antibody and the protein and the excess of unreacted gold particles.

## Claims

1. Methods for quantitative determination of human maternal milk using the dosage of Phospho-perilipin (p-LAP) as a biomarker.

2. Methods as in claim 1 where Phospho-Perilina (p-LAP) are phosphorylated proteins belonging to the class of proteins identified by National Center for Biotechnology Information (NCBI) as Enterez GeneID: 5346 perilipin 1; 123 perilipin 2; 10226 perilipin 3; 729359 perilipin 4; 440503 perilipin 5.

3. Methods according to the preceding claims, wherein the quantitative assay of Phospho-Perilina (p-LAP) and / or its isomers or its chemical and / or biological derivatives, are immunoassay of serine 522 p-LAP, serine 497 p-LAP.

4. Methods according to the previous claims combined with the use of microRNAs as: miR16, miR21, miR146a, miR155.

5. Methods according to the preceding claims combined with assay of casein and whey protein.

6. Methods according to the preceding claims using a device consisting of a support strip for the immunoassay, coated with antibodies and/or aptamer suitable for the purpose and associated with colloidal gold particles able to reacting with the biomarkers

7. Methods according to the preceding claims, wherein the biomarkers are Phospho-Perilipin (p-LAP); microRNA; casein, whey proteins.

8. Methods according to the preceding claims where: the anti-Phospho-Perilipin antibody (p-LAP) on a support of the nitrocellulose or other suitable agent, colloidal gold particles.

9. Methods according to the preceding claims where the device comprising: a plastic support strip (stick), an anti-Phospho-Perilipin antibody (p-LAP) with a molecular weight between 35kDa and 75kDa and preferably 55kDa, a support of nitrocellulose or other suitable agent have a porosity comprised of between 0.25 and 0.95 nm, and preferably 0.45 nm, colloidal gold particles having a size of between 0.3 and 0.9 nm, and preferably 0.60 nm

10. Methods according to the preceding claims where: the anti-Phospho-Perilipin aptamer (p-LAP) on a support of nitrocellulose or other suitable agent, colloidal gold particles.
